# EUROPÄISCHE PATENTSCHRIFT

(11) **EP 2 507 204 B1**
(45) Veröffentlichungstag und Bekanntmachung des Hinweises auf die Patenterteilung: **06.08.2014**
(21) Anmeldenummer: 10787099.0
(22) Anmeldetag: 01.12.2010
(51) Int. Cl.: C07C 231/02, C07C 233/03, C07C 233/43

(54) **VERFAHREN ZUR HERSTELLUNG VON AROMATISCHEN FORMAMIDEN**
METHOD FOR PRODUCING AROMATIC FORMAMIDES
PROCÉDÉ DE FABRICATION DE FORMAMIDES AROMATIQUES

(30) Priorität: 04.12.2009 EP 09178057
(43) Veröffentlichungstag der Anmeldung: 10.10.2012
(73) Patentinhaber: BASF SE, 67056 Ludwigshafen (DE)
(72) Erfinder: FRANZKE, Axel, 68161 Mannheim (DE); MATTKE, Torsten, 67251 Freinsheim (DE); LESCHINSKI, Julia, 68161 Mannheim (DE); ABDALLAH, Radwan, 67063 Ludwigshafen (DE); BOCK, Michael, 67152 Ruppertsberg (DE); BAUMANN, Robert, 68529 Mannheim (DE); STRÖFER, Eckhard, 68163 Mannheim (DE)
(86) Internationale Anmeldenummer: PCT/EP2010/068621
(87) Internationale Veröffentlichungsnummer: WO 2011/067278

(56) Entgegenhaltungen:
- US-A- 4 258 200
- DASZKIEWICZ, Z. ET AL: "A simple route to N-methylarylamines" CHEMICAL PAPERS, Bd. 47, Nr. 2, 1993, Seiten 109-113, XP008132451 in der Anmeldung erwähnt
- LEWIN, ANITA H. ET AL: "Galanthamine analogs: 6H-benzofuro[3a,3,2,-e,f][1]benzazepine and 6H-benzofuro[3a,3,2-e,f][3]benzazepine" TETRAHEDRON, Bd. 61, Nr. 30, 2005, Seiten 7144-7152, XP002619342 in der Anmeldung erwähnt
- ISHII, YASUTAKA ET AL: "Acylation of Alcohols and Amines with Vinyl Acetates Catalyzed by Cp2Sm(thf)2" JOURNAL OF ORGANIC CHEMISTRY, Bd. 61, Nr. 9, 1996, Seiten 3088-3092, XP002619343 in der Anmeldung erwähnt

## Beschreibung

Gegenstand der Erfindung ist ein Verfahren zur Herstellung von aromatischen Formamiden durch Umsetzung von mono-, di- oder polyfunktionalen aromatischen Aminen mit einem Ameisensäureester in Gegenwart katalytischer Mengen einer phosphorhaltigen Säure oder eines Lewis-sauren Zink, Blei- oder Ytterbium salzes, einer anorganischen oder organischen Säure wobei bereits nach kurzen Reaktionszeiten hohe Selektivitäten und Ausbeuten erzielt werden. Die so hergestellten Formamide können in weiterer Folge zu technisch bedeutsamen Isocyanaten umgesetzt werden.

Die thermische Umsetzung von aliphatischen Aminen mit Methylformiat zu den entsprechenden Formamiden kann mit sehr guten Selektivitäten und Raum-Zeit-Ausbeuten durchgeführt werden und wird auch im großtechnischen Maßstab, beispielsweise zur Produktion von N,N-Dimethylformamid (DMF), angewendet (Industrielle Organische Chemie, Wiley-VCH, Weinheim, 2007 (6), 49). Eine analoge Transformation von aromatischen Aminen zu den jeweiligen Formamiden ist wegen der verringerten Nukleophilie dieser Amine hingegen deutlich weniger effizient. Hierbei werden selbst nach langen Reaktionszeiten nur unbefriedigende Umsätze und Ausbeuten erreicht, die einen potenziellen industriellen Einsatz dieser Verfahren erschweren (beispielsweise DE 3832571, Journal of Organic Chemistry 1966, (31), 3473-3482 und Tetrahedron 2004, (60), 81-92).

Um diesen Nachteil auszugleichen, wurden in der Vergangenheit einige alternative Verfahren zur Herstellung von aromatischen Formamiden beschrieben. So liefert die Umsetzung von aromatischen Aminen mit einem Überschuss an Ameisensäure die entsprechenden Formamide in hohen Ausbeuten (DE 138839 und Bulletin of the Korean Chemical Society 2002, (23), 149-150). Allerdings müssen hierbei wegen der erheblichen Korrosivität der Ameisensäure die Apparate aus höherwertigen und damit teureren Werkstoffen gefertigt werden. Weiterhin wird die Ameisensäure großtechnisch durch saure Hydrolyse von Methylformiat gewonnen. Eine direkte Verwendung des Methylformiates würde somit eine effizientere und kostengünstigere Syntheseroute darstellen.

Die Verwendung von Reaktivestern der Ameisensäure mit beispielsweise 2,2,2-Trifluorethanol oder Pentafluorphenol ermöglicht hohe Ausbeuten unter milden Bedingungen (Synthesis 1987, 510 und Organic Letters 2002, (4), 111-113). Diese Reagenzien sind für industrielle Anwendungen jedoch nur sehr eingeschränkt einsetzbar, da sie sowohl teuer als auch nicht in großen Mengen verfügbar sind.

Der stöchiometrische Einsatz von starken Basen wie Natriumhydrid sowie Lithiumhexamethyldisilazid oder auf Kieselgel geträgertem Phosphortrichlorid führt ebenfalls zu effizienten Umsetzungen (Organic Letters 2009, (11), 389-892, Organic Letters 2007, (9), 3631-3634 und Tetrahedron Letters 2005, (46), 7963-7966). Hierbei wird jedoch eine große Menge an Nebenprodukten generiert, die entsorgt oder teuer rückgeführt werden muss.

Es wurden bisher nur wenige Synthesen von Formamiden aus aromatischen Aminen und Ameisensäurealkylestern in der Gegenwart von katalytisch aktiven Verbindungen beschrieben. Hierbei wurden zum einen Säuren wie para-Toluolsulfonsäure, Trifluoressigsäure oder geringe Mengen an Ameisensäure verwendet (Organic Letters 2006, (8), 1875-1878, Tetrahedron 2005, (61), 7144-7152, Journal of Organic Chemistry 1966, (31), 3473-3482 und Chemical Papers 1993, (47), 109-113). Daszkiewicz et al. (Chemical Papers 1993, (47), 109-113) beschreibt zum Beispiel die Herstellung von aromatischen substituierten Formaniliden durch Reaktion von ringsubstituierten Anilinen mit n-Butylformiat in Gegenwart von Trifluoressigsäure als Katalysator. Der Einsatz von Natriummethoxid oder Samarocenen ist ebenfalls beschrieben worden (US 2005/0027120 und Journal of Organic Chemistry 1996, (61), 3088-3092). Aber auch in diesen Fällen sind entweder die Ausbeuten für eine großtechnische Anwendung zu niedrig, es werden teure höhere Alkylformiate verwendet und/oder die Katalysatoren sind recht kostspielig oder korrosiv.

US 4 258 200 beschreibt die Herstellung von Säureamiden mit Cobaltkatalysatoren.

Der Erfindung lag die Aufgabe zu Grunde, ein industriell durchführbares Verfahren zur Herstellung von Formamiden aus mono-, di- oder polyfunktionalen aromatischen Aminen zu entwickeln, welches auch unter Verwendung großtechnisch zugänglicher Ameisensäureester in Gegenwart eines Katalysators hohe Raum-Zeit-Ausbeuten und Selektivitäten ermöglicht.

Überraschenderweise hat sich gezeigt, dass durch Umsetzung von aromatischen Aminen mit einem Alkylformiat in Gegenwart einer phosphorhaltigen Säure oder eines Lewis-sauren Zink, Blei- oder Ytterbium salzes einer anorganischen oder organischen Säure die erwunschten Formamide schon nach kurzen Reaktions-zeiten in sehr guten Ausbeuten isoliert werden.

Gegenstand der Erfindung ist ein Verfahren zur Herstellung von Formamiden durch Umsetzung von aromatischen Aminen mit einem Ameisensäureester in Gegenwart eines Katalysators, dadurch gekennzeichnet, dass der Katalysator eine phosphorhaltige Säure oder ein Lewis-saures Metallsalz einer anorganischen oder organischen Säure ist und das Metall des Lewis-sauren Metallsalzes ausgewählt ist aus der Gruppe enthaltend: Zink, Blei und Ytterbium.

Beispiele für die bei dem erfindungsgemäßen Verfahren eingesetzten phosphorhaltigen Säuren (= Protonen-Donatoren) sind Phosphor(III)säuren, wie C₁-C₁₀-Alkyl-, bevorzugt C₁-C₄-Alkyl, oder C₆-C₁₄-Aryl-, bevorzugt C₆-C₁₀-Arylphosphonsäuren, gegebenenfalls an einer polymeren Festphase geträgert, und Phosphor(V)säuren wie ortho-Phosphorsäure oder deren höhere Kondensate (Diphosphorsäure, Metaphosphorsäure oder Polyphosphorsäure). Bei den vorgenannten Phosphorsäuren kann ein Teil der Säurefunktionen mit niederen C₁-C₄-Alkoholen, wie beispielsweise Methanol, Ethanol oder n-Butanol, verestert sein. Diese phosphorhaltigen Säuren können in reiner Form oder als Gemisch eingesetzt werden. Besonders bevorzugt wird ortho-Phosphorsäure in wasserfreier oder wässriger Form eingesetzt.

Die bei dem erfindungsgemäßen Verfahren eingesetzten Lewis-sauren Metallsalze (= Elektronenpaar-Akzeptoren) sind Salze ausgewählt aus der Gruppe von: Zink, Blei, oder Ytterbium. Bei den vorgenannten Metallsalzen handelt es sich im Allgemeinen um die entsprechenden Fluoride, Chloride, Sulfate, Nitrate, Phosphate, Carboxylate oder Sulfonate.

Bei den Carboxylaten handelt es sich im Allgemeinen um die durch Deprotonierung hervorgegangenen Anionen von Carbonsäuren der allgemeinen Formel R(CO₂H)ₙ. R bezeichnet hierbei C₁-C₁₈-Alkylreste, C₂-C₇-Alkenylreste, bevorzugt Ethenyl, C₅-C₈-Cycloalkylreste, aromatische C₆-C₁₄-Arylreste, bevorzugt Phenyl oder Naphthyl, oder Reste der Naphthensäuren der allgemeinen Struktur (I) 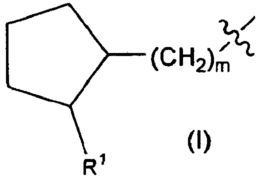 worin R' einen Wasserstoff- oder Methylencyclopentylrest und m Null oder eine ganze Zahl von 1 bis 12 bezeichnet,
und n eine ganze Zahl von 1 bis 4. Vorzugsweise ist n gleich 1.

Bevorzugt sind Carboxylate, worin R für einen C₁-C₁₀-Alkylrest, insbesondere C₁-C₆-Alkylrest, C₅-C₈-Cycloalkylrest oder für einen Naphthensäurerest der obigen Struktur (I) steht. Besonders bevorzugt bezeichnet R die Reste Methyl, Ethyl, Propyl oder Cyclopentyl. Alle genannten Carboxylate können einzeln oder als Mischung verwendet werden.

Unter den Sulfonaten sind im Allgemeinen gegebenenfalls substituierte C₁-C₄-Alkyl-, insbesondere Methyl- oder Ethyl-, C₆-C₁₀-Aryl-, insbesondere Phenyl- oder Tolyl-, oder C₁₀-C₁₄-Alkylbenzolsulfonate zu verstehen. Die vorgenannten Alkylreste sind vorzugsweise durch Halogen, insbesondere Fluor, einfach oder mehrfach substituiert. Besonders bevorzugt ist Trifluormethansulfonat.

Die beschriebenen Lewis-sauren Metallsalze umfassen auch die entsprechenden Mono-, Di- oder Polyhydrate mit Kristallwasser.

Die Carboxylate, Sulfonate oder Nitrate von Zink, Blei oder Ytterbium, sind besonders bevorzugt. Ganz besonders bevorzugt sind Zinkacetat, Zinkacetat-Dihydrat, Zinknaphthenat oder Ytterbiumtrifluormethansulfonat.

Der Katalysator wird in einem molaren Verhältnis von 0,001 bis 0,3, bevorzugt von 0,01 bis 0,1, jeweils bezogen auf die Aminogruppen eingesetzt.

Der Ameisensäureester leitet sich von einem linearen oder verzweigten aliphatischen Alkohol mit 1 bis 6 Kohlenstoffatomen, wie Methanol, Ethanol, n-Propanol, n-Butanol, n-Pentanol oder n-Hexanol, oder von einem linearen oder verzweigten 1-Alkenylformiat mit 2-6 Kohlenstoffatomen im Alkenylrest, wie Vinylformiat oder Isoprenylformiat, ab. Die vorgenannten Ameisensäureester können einzeln oder als Mischung verwendet werden. Bevorzugt werden lineare oder verzweigte C₁-C₆-Alkylformiate verwendet, besonders bevorzugt Methylformiat, das auch im großtechnischen Maßstab verfügbar ist. Methylformiat wird vorzugsweise durch Umsetzung von Kohlenmonoxid mit Methanol hergestellt.

Der Ameisensäureester wird in einem molaren Verhältnis Ameisensäureester zu Aminogruppen von 1:1 bis 20:1, bevorzugt von 1.5:1 bis 8:1, eingesetzt.

Die Umsetzung des aromatischen Amins mit dem Ameisensäureester in Gegenwart des Katalysators wird vorzugsweise bei einer Reaktionstemperatur von 20-160°C, besonders bevorzugt von 60-120°C, durchgeführt. Bei dieser Temperatur wird innerhalb von 0.5-5 h ein quantitativer Umsatz des aromatischen Amins erhalten, wobei Reaktionszeiten von 2 h routinemäßig erreicht werden. Die Druckbedingungen werden im Allgemeinen in Abhängigkeit von dem verwendeten Ameisensäureester beziehungsweise dessen Siedetemperatur ausgewählt. Die Umsetzung kann bei autogenem Druck (Druck, der sich bei der Reaktion im geschlossenen Gefäß bei der Reaktionstemperatur einstellt) oder aber auch einem höheren Druck von 1 bis 100 bar absolut oder einem Unterdruck von 0.001 bis 1 bar absolut durchgeführt werden. Als Lösungsmittel können der Ameisensäureester selbst oder andere inerte Verbindungen verwendet werden. Geeignete Lösungsmittel sind beispielsweise Amide wie N,N-Dimethylformamid oder N,N-Dimethylacetamid, Sulfoxide wie Dimethylsulfoxid, aromatische Kohlenwasserstoffe mit oder ohne Alkyl-, Halogen- oder Alkoxysubstituenten wie Toluol, die isomeren Xylole, Mesitylen, Ethylbenzol, Chlorbenzol, die isomeren Dichlor- oder Trichlorbenzole, Anisol, Mono- oder Polyether wie Tetrahydrofuran, Dioxan oder Dimethoxyethan, Dialkylethylenglykole wie beispielsweise Diethylenglykoldimethylether und Triethylenglykoldimethylether. Diese können einzeln oder als Gemisch eingesetzt werden.

Bei dem erfindungsgemäßen Verfahren werden mono-, di- oder polyfunktionale aromatische Amine eingesetzt. Die vorgenannten Amine sind primäre oder sekundäre Amine der allgemeinen Formel R²(NHR³)ₙ, worin R² für einen gegebenenfalls substituierten C₆-C₃₄-Arylrest, vorzugsweise C₆-C₂₀-Arylrest, insbesondere C₆-C₁₄-Arylrest steht, und besonders bevorzugt Phenyl oder Tolyl bzw. Toluylen ist. R³ steht für einen C₁-C₄-Alkylrest, insbesondere Methyl- oder Ethylrest, oder ein Wasserstoffatom und n ist eine ganze Zahl von 1 bis 3, vorzugsweise 1 oder 2, pro aromatischem Zyklus. Bevorzugt ist R³ ein Wasserstoffatom. Geeignete Substituenten des Arylrests sind zum Beispiel Chlor, Fluor, Brom, Cyano, Alkyl, Alkoxy, Alkylcarbonyl und/oder Alkoxycarbonyl, wobei Alkyl und Alkoxy im Allgemeinen 1 bis 10, vorzugsweise 1 bis 6, besonders bevorzugt 1 bis 4 Kohlenstoffatome, aufweisen. Vertreter aus dieser Gruppe sind beispielsweise Anilin, o-, m- und/oder p-Toluidin, o-, m- und/oder p-Chloranilin, o-, m- und/oder p-Bromanilin, o-, m- und/oder p-Trifluormethylanilin, 2,4-, 2,6-, 3,4- und/oder 3,5-Dimethyl-, -Dichlor-, -Dibrom- oder -Diethylanilin, p-tert-Butylanilin, Diaminotoluol (TDA), insbesondere 2,4- und/oder 2,6-Diaminotoluol, Diaminodiphenylmethan (MDA), insbesondere 2,4'-Diaminodiphenylmethan, 4,4'-Diaminodiphenylmethan, 2,2'-Diaminodiphenylmethan und/oder höhere Homologe (Polyphenylenpolymethylenpolyamine) oder o-, m- und/oder p-Phenylendiamin. Bevorzugt werden Anilin, die Isomeren von Diaminotoluol, insbesondere 2,4- und 2,6- Diaminotoluol, und/oder die Isomeren und höheren Homologen von Diaminodiphenylmethan eingesetzt.

Die Isolierung der aromatischen Formamide kann auf verschiedenen, dem Fachmann vertrauten Wegen erfolgen. Hierbei kann es sich beispielsweise um eine fraktionierte Destillation handeln. Alternativ kann der Reaktionsaustrag bis zur Trockene eingeengt werden und der hieraus resultierende Feststoff durch Waschen mit oder Umkristallisation aus einem geeigneten Lösungsmittel aufgereinigt werden. Weiterhin kann das Wertprodukt durch Zugabe eines geeigneten Lösungsmittels gefällt, durch Filtration isoliert und durch Waschen oder Umkristallisation aufgereinigt werden

In einer bevorzugten Ausführungsform des erfindungsgemäßen Verfahrens zur Herstellung von Formamiden fällt das Wertprodukt schon während oder erst nach beendeter Reaktion als in überschüssigem Reagenz oder Lösungsmittel suspendierter Feststoff an, der dann durch Filtration isoliert wird. Diese kann bei Reaktionstemperatur oder auch nach entsprechender Abkühlung auf beispielsweise Raumtemperatur unter autogenem Druck, Normaldruck oder auch höherem Druck erfolgen. Das Formamid fällt hierbei typischerweise in für die weitere Verarbeitung ausreichend reiner Form an, kann nötigenfalls aber auch durch Waschen mit Ameisensäureester oder Lösungsmittel weiter aufgereinigt werden. Nach der Trennung des Feststoffes vom Filtrat kann ersterer in fester Form, als Schmelze oder auch nach Auflösung in einem geeigneten Solvens isoliert und gegebenenfalls einer weiteren Reaktionsstufe zugeführt werden. Aus dem Filtrat können das überschüssige Reagenz, das eventuell in der Reaktion verwendete Lösungsmittel und der Katalysator einzeln oder getrennt isoliert und in einem weiteren Reaktionszyklus verwendet werden. Auch eventuell vorhandene Reste an nicht umgesetztem Edukt und/oder der bei der Umsetzung von Polyaminen anfallenden Intermediate (Aminoformamide) können so isoliert und in das Verfahren rückgeführt werden. Das Verfahren kann sowohl kontinuierlich als auch diskontinuierlich durchgeführt werden.

Die gewonnenen Formamide können, wenn sie sich von primären aromatischen Aminen ableiten, zu technisch wichtigen aromatischen Isocyanaten verarbeitet werden beispielsweise durch oxidative Dehydrierung.

Die Erfindung wird in den nachstehenden Beispielen näher erläutert ohne diese darauf zu beschränken.

### Beispiele

### Beispiel 1

10.0 g (81.9 mmol) 2,4-Diaminotoluol wurde in 40.0 g N,N-Dimethylacetamid und 49.2 g (819 mmol) Methylformiat gelöst und mit 0.47 g (4.1 mmol) 85 Gew.-%iger wässriger ortho-Phosphorsäure versetzt. Das Gemisch wurde in einen 300 mL-Autoklaven gefüllt und 4 h bei 90°C unter autogenem Druck gerührt. Nach dem Abkühlen auf Raumtemperatur wurde die resultierende Mischung bis zur Trockene eingeengt. Der erhaltene bräunliche Feststoff (17.3 g) enthielt neben Lösungsmittelresten und dem Katalysator laut NMR-Spektroskopie das Bisformamid und die regioisomeren Monoamide in einem molaren Verhältnis von 97:3.

### Beispiel 2

10.0 g (81.9 mmol) 2,4-Diaminotoluol wurde in 40.0 g N,N-Dimethylacetamid und 49.2 g (819 mmol) Methylformiat gelöst und mit 2.54 g (4.1 mmol) Ytterbium(III)trifluormethansulfonat versetzt. Das Gemisch wurde in einen 300 mL-Autoklaven gefüllt und 4 h bei 90°C unter autogenem Druck gerührt. Nach dem Abkühlen auf Raumtemperatur wurde die resultierende Mischung bis zur Trockene eingeengt. Der erhaltene bräunliche Feststoff (23.4 g) enthielt neben Lösungsmittelresten und dem Katalysator laut NMR-Spektroskopie das Bisformamid und die regioisomeren Monoamide in einem molaren Verhältnis von 94:6.

### Beispiel 3

10.0 g (81.9 mmol) 2,4-Diaminotoluol wurde in 40.0 g N,N-Dimethylacetamid und 49.2 g (819 mmol) Methylformiat gelöst und mit 0.75 g (4.1 mmol) Zink(II)acetat versetzt. Das Gemisch wurde in einen 300 mL-Autoklaven gefüllt und 4 h bei 90°C unter autogenem Druck gerührt. Nach dem Abkühlen auf Raumtemperatur wurde die resultierende Mischung bis zur Trockene eingeengt. Der erhaltene bräunliche Feststoff (16.4 g) enthielt neben Lösungsmittelresten und dem Katalysator laut NMR-Spektroskopie das Bisformamid und die regioisomeren Monoamide in einem molaren Verhältnis von 92:8.

### Beispiel 4

10.0 g (81.9 mmol) 2,4-Diaminotoluol wurde in 40.0 g N,N-Dimethylacetamid und 49.2 g (819 mmol) Methylformiat gelöst und mit 1.55 g (4.1 mmol) Blei(II)acetat versetzt. Das Gemisch wurde in einen 300 mL-Autoklaven gefüllt und 4 h bei 90°C unter autogenem Druck gerührt. Nach dem Abkühlen auf Raumtemperatur wurde die resultierende Mischung bis zur Trockene eingeengt. Der erhaltene bräunliche Feststoff (15.7 g) enthielt neben Lösungsmittelresten und dem Katalysator laut NMR-Spektroskopie das Bisformamid und die regioisomeren Monoamide in einem molaren Verhältnis von 73:27.

### Vergleichsbeispiel 1

10.0 g (81.9 mmol) 2,4-Diaminotoluol wurde in 40.0 g N,N-Dimethylacetamid und 49.2 g (819 mmol) Methylformiat gelöst und mit 0.40 g (4.2 mmol) Methansulfonsäure versetzt. Das Gemisch wurde in einen 300 mL-Autoklaven gefüllt und 4 h bei 90°C unter autogenem Druck gerührt. Nach dem Abkühlen auf Raumtemperatur wurde die resultierende Mischung bis zur Trockene eingeengt. Das erhaltene braune, hochviskose Öl (14.8 g) enthielt neben Lösungsmittelresten und dem Katalysator laut NMR-Spektroskopie das Bisformamid, die regioisomeren Monoamide und 2,4-Diaminotoluol in einem molaren Verhältnis von 10:75:15.

### Vergleichsbeispiel 2

10.0 g (81.9 mmol) 2,4-Diaminotoluol wurde in 40.0 g N,N-Dimethylacetamid und 49.2 g (819 mmol) Methylformiat gelöst und mit 0.42 g (4.1 mmol) 96 Gew.-%iger wässriger Schwefelsäure versetzt. Das Gemisch wurde in einen 300 mL-Autoklaven gefüllt und 4 h bei 90°C unter autogenem Druck gerührt. Nach dem Abkühlen auf Raumtemperatur wurde die resultierende Mischung bis zur Trockene eingeengt. Das erhaltene braune, hochviskose Öl (15.2 g) enthielt neben Lösungsmittelresten und dem Katalysator laut NMR-Spektroskopie das Bisformamid, die regioisomeren Monoamide und 2,4-Diaminotoluol in einem molaren Verhältnis von 23:74:3.

### Vergleichsbeispiel 3

10.0 g (81.9 mmol) 2,4-Diaminotoluol wurde in 40.0 g N,N-Dimethylacetamid und 49.2 g (819 mmol) Methylformiat gelöst und mit 0.65 g (4.1 mmol) Phenylsulfonsäure versetzt. Das Gemisch wurde in einen 300 mL-Autoklaven gefüllt und 4 h bei 90°C unter autogenem Druck gerührt. Nach dem Abkühlen auf Raumtemperatur wurde die resultierende Mischung bis zur Trockene eingeengt. Das erhaltene braune, hochviskose Öl (15.6 g) enthielt neben Lösungsmittelresten und dem Katalysator laut NMR-Spektroskopie das Bisformamid, die regioisomeren Monoamide und 2,4-Diaminotoluol in einem molaren Verhältnis von 12:76:12.

### Vergleichsbeispiel 4

10.0 g (81.9 mmol) 2,4-Diaminotoluol wurde in 40.0 g N,N-Dimethylacetamid und 49.2 g (819 mmol) Methylformiat gelöst und mit 1.57 g (4.1 mmol) Zirkonium(IV)-tert-butoxid versetzt. Das Gemisch wurde in einen 300 mL-Autoklaven gefüllt und 4 h bei 90°C unter autogenem Druck gerührt. Nach dem Abkühlen auf Raumtemperatur wurde die resultierende Mischung bis zur Trockene eingeengt. Das erhaltene braune, hochviskose Öl (15.4 g) enthielt neben Lösungsmittelresten und dem Katalysator laut NMR-Spektroskopie das Bisformamid, die regioisomeren Monoamide und 2,4-Diaminotoluol in einem molaren Verhältnis von 41:58:1.

### Vergleichsbeispiel 5

10.0 g (81.9 mmol) 2,4-Diaminotoluol wurde in 40.0 g N,N-Dimethylacetamid und 49.2 g (819 mmol) Methylformiat gelöst. Das Gemisch wurde ohne Katalysator in einen 300 mL-Autoklaven gefüllt und 12 h bei 90°C unter autogenem Druck gerührt. Nach dem Abkühlen auf Raumtemperatur wurde die resultierende Mischung bis zur Trockene eingeengt. Das erhaltene braune, hochviskose Öl enthielt neben Lösungsmittelresten laut NMR-Spektroskopie und Dünnschichtchromatographie keine signifikanten Mengen an Bisformamid, sondern lediglich die regioisomeren Monoamide und 2,4-Diaminotoluol.

### Beispiel 5

10.0 g (81.9 mmol) 2,6-Diaminotoluol wurde in 40.0 g N,N-Dimethylacetamid und 49.2 g (819 mmol) Methylformiat gelöst und mit 0.47 g (4.1 mmol) 85 Gew.-%iger wässriger ortho-Phosphorsäure versetzt. Das Gemisch wurde in einen 300 mL-Autoklaven gefüllt und 4 h bei 90°C unter autogenem Druck gerührt. Nach dem Abkühlen auf Raumtemperatur wurde die resultierende Mischung bis zur Trockene eingeengt. Der erhaltene bräunliche Feststoff (14.6 g) enthielt neben Lösungsmittelresten und dem Katalysator laut NMR-Spektroskopie das Bisformamid und das Monoamid in einem molaren Verhältnis von 97:3.

### Beispiel 6

21.7 g (178 mmol) 2,4-Diaminotoluol wurde in 21.7 g N,N-Dimethylacetamid und 107 g (1.78 mol) Methylformiat gelöst und mit 1.02 g (8.8 mmol) 85 Gew.-%iger wässriger ortho-Phosphorsäure versetzt. Das Gemisch wurde in einen 300 mL-Autoklaven gefüllt und 2 h bei 90 °C unter autogenem Druck gerührt. Nach dem Abkühlen auf Raumtemperatur wurde die resultierende Suspension mit 70 g Methylformiat verdünnt, filtriert und der fast farblose Feststoff mit 70 g Methylformiat gewaschen und getrocknet. Auf diese Art und Weise wurde 29.6 g (94%) laut Dünnschichtchromatographie und NMR-Spektroskopie reines Bisformamid isoliert.

### Beispiel 7

21.7 g (178 mmol) 2,4-Diaminotoluol wurde in 128 g (2.13 mol) Methylformiat gelöst und mit 1.63 g (8.9 mmol) Zink(II)acetat versetzt. Das Gemisch wurde in einen 300 mL-Autoklaven gefüllt und 2 h bei 90°C unter autogenem Druck gerührt. Nach dem Abkühlen auf Raumtemperatur wurde die resultierende Suspension mit 70 g Methylformiat verdünnt, filtriert und der fast farblose Feststoff mit 70 g Methylformiat gewaschen und getrocknet. Auf diese Art und Weise wurde 29.8 g (94%) laut Dünnschichtchromatographie und NMR-Spektroskopie reines Bisformamid isoliert.

### Beispiel 8

20.0 g (164 mmol) 2,6-Diaminotoluol wurde in 118 g (1.97 mol) Methylformiat gelöst und mit 5.01 g (8.0 mmol) 65 Gew.-%igem Zink(II)naphthenat in Mineralöl (entspricht einer 10 Gew.-%igen Zinklösung) versetzt. Das Gemisch wurde in einen 300 mL-Autoklaven gefüllt und 2 h bei 90°C unter autogenem Druck gerührt. Nach dem Abkühlen auf Raumtemperatur wurde die resultierende Suspension mit 70 g Methylformiat verdünnt, filtriert und der fast farblose Feststoff mit 70 g Methylformiat gewaschen und getrocknet. Auf diese Art und Weise wurde 26.7 g (92%) laut Dünnschichtchromatographie und NMR-Spektroskopie reines Bisformamid isoliert.

### Beispiel 9

21.7 g (178 mmol) 2,4-Diaminotoluol und 2,6-Diaminotoluol im Verhältnis 80:20 wurden in 128 g (2.13 mol) Methylformiat gelöst und mit 1.02 g (8.8 mmol) 85 Gew.-%iger wässriger ortho-Phosphorsäure versetzt. Das Gemisch wurde in einen 300 mL-Autoklaven gefüllt und 2 h bei 90°C unter autogenem Druck gerührt. Nach dem Abkühlen auf Raumtemperatur wurde die resultierende Suspension mit 70 g Methylformiat verdünnt, filtriert und der fast farblose Feststoff mit 70 g Methylformiat gewaschen und getrocknet. Auf diese Art und Weise wurde 27.8 g (88%) laut Dünnschichtchromatographie und NMR-Spektroskopie reine Mischung der regioisomeren Bisformamide isoliert.

### Beispiel 10

15.0 g (161 mmol) Anilin wurde in 60.0 g N,N-Dimethylacetamid und 48.5 g (808 mmol) Methylformiat gelöst und mit 1.07 g (4.9 mmol) Zink(II)acetat-Dihydrat versetzt. Das Gemisch wurde in einen 300 mL-Autoklaven gefüllt und 2 h bei 90°C unter autogenem Druck gerührt. Nach dem Abkühlen auf Raumtemperatur wurden der Überschuss an Methylformiat und das entstandene Methanol abdestilliert. Das erhaltene rotbraune Öl enthielt neben größeren Mengen an Lösungsmittel und dem Katalysator laut NMR-Spektroskopie lediglich Formanilid und Anilin in einem molaren Verhältnis von 99:1.

### Beispiel 11

15.0 g (161 mmol) Anilin wurde in 60.0 g N,N-Dimethylacetamid und 48.5 g (808 mmol) Methylformiat gelöst und mit 0.56 g (4.9 mmol) 85 Gew.-%iger wässriger ortho-Phosphorsäure versetzt. Das Gemisch wurde in einen 300 mL-Autoklaven gefüllt und 2 h bei 90°C unter autogenem Druck gerührt. Nach dem Abkühlen auf Raumtemperatur wurden der Überschuss an Methylformiat und das entstandene Methanol abdestilliert. Das erhaltene rotbraune Öl enthielt neben größeren Mengen an Lösungsmittel und dem Katalysator laut NMR-Spektroskopie lediglich Formanilid und Anilin in einem molaren Verhältnis von 98:2.

Die Beispiele zeigen, dass durch das erfindungsgemäße Verfahren aromatische Formamide in hohen Selektivitäten und hohen Raum-Zeit-Ausbeuten erhältlich sind. Die Produkte werden in hoher Reinheit gebildet, so dass keine aufwändige Nachreinigung erforderlich ist.

## Patentansprüche

1. Verfahren zur Herstellung von Formamiden durch Umsetzung von aromatischen Aminen mit einem Ameisensäureester in Gegenwart eines Katalysators, **dadurch gekennzeichnet, dass** der Katalysator eine phosphorhaltige Säure oder ein Lewis-saures Metallsalz einer anorganischen oder organischen Säure ist und das Metall des Lewis-sauren Metallsalzes ausgewählt ist aus der Gruppe enthaltend: Zink, Blei und Ytterbium.

2. Verfahren nach Anspruch 1, **dadurch gekennzeichnet, dass** die phosphorhaltige Säure ortho-Phosphorsäure oder eines ihrer höhermolekularen Kondensate ist.

3. Verfahren nach Anspruch 1, **dadurch gekennzeichnet, dass** das Lewis-saure Metallsalz ein Carboxylat, Sulfonat oder Nitrat von Zink, Blei oder Ytterbium ist.

4. Verfahren nach Anspruch 3, **dadurch gekennzeichnet, dass** das Lewis-saure Metallsalz Zinkacetat, Zinkacetat-Dihydrat, Zinknaphthenat oder Ytterbiumtrifluormethansulfonat ist.

5. Verfahren nach einem der Ansprüche 1 bis 4, **dadurch gekennzeichnet, dass** der Katalysator in einem molaren Verhältnis von 0,001 bis 0,3 bezogen auf die Aminogruppen eingesetzt wird.

6. Verfahren nach einem der Ansprüche 1 bis 5, **dadurch gekennzeichnet, dass** der Ameisensäureester ein lineares oder verzweigtes C₁-C₆-Alkyl- oder C₂-C₆-1-Alkenylformiat ist.

7. Verfahren nach Anspruch 6, **dadurch gekennzeichnet, dass** der Ameisensäureester Methylformiat ist.

8. Verfahren nach einem der Ansprüche 1 bis 7, **dadurch gekennzeichnet, dass** der Ameisensäureester in einem molaren Verhältnis Ameisensäureester zu Aminogruppen von 1:1 bis 20:1 eingesetzt wird.

9. Verfahren nach einem der Ansprüche 1 bis 8, **dadurch gekennzeichnet, dass** die aromatischen Amine primäre oder sekundäre Amine der allgemeinen Formel R²(NHR³)ₙ sind, worin R für einen gegebenenfalls substituierten C₆-C₃₄-Arylrest steht, R³ ein C₁-C₄-Alkylrest oder ein Wasserstoffatom ist und n eine ganze Zahl von 1 bis 3 pro aromatischem Zyklus ist.

10. Verfahren nach Anspruch 9, **dadurch gekennzeichnet, dass** die aromatischen Amine primäre Amine sind.

11. Verfahren nach Anspruch 10, **dadurch gekennzeichnet, dass** die aromatischen Amine ausgewählt sind aus der Gruppe enthaltend: Anilin, Diaminotoluol (TDA), insbesondere 2,4- und 2,6-Diaminotoluol und deren Isomerengemische, Diaminodiphenylmethan (MDA), insbesondere 2,4'-Diaminodiphenylmethan, 4,4'-Diaminodiphenylmethan, 2,2'-Diaminodiphenylmethan und höhere Homologe (Polyphenylenpolymethylenpolyamine) und deren Gemische.

12. Verfahren nach einem der Ansprüche 1 bis 11, **dadurch gekennzeichnet, dass** der suspensionsförmige Reaktionsaustrag filtriert wird, wobei das Formamid als Feststoff isoliert wird.

13. Verfahren nach Anspruch 12, **dadurch gekennzeichnet, dass** der im Filtrat befindliche Katalysator und gegebenenfalls vorhandene Lösungsmittel, Überschüsse an Ameisensäureester, Eduktreste und Aminoformamide einzeln oder getrennt in einem weiteren Reaktionszyklus wieder verwendet beziehungsweise rückgeführt werden.

## Claims

1. A process for the preparation of formamides by reacting aromatic amines with a formic acid ester in the presence of a catalyst, wherein the catalyst is a phosphorus-containing acid or a Lewis-acidic metal salt of an inorganic or organic acid and the metal of the Lewis-acidic metal salt is selected from the group comprising: zinc, lead and ytterbium.

2. The process according to claim 1, wherein the phosphorus-containing acid is ortho-phosphoric acid or one of its relatively high-molecular weight condensates.

3. The process according to claim 1, wherein the Lewis-acidic metal salt is a carboxylate, sulfonate or nitrate of zinc, lead or ytterbium.

4. The process according to claim 3, wherein the Lewis-acidic metal salt is zinc acetate, zinc acetate dihydrate, zinc naphthenate or ytterbium trifluoromethanesulfonate.

5. The process according to any one of claims 1 to 4, wherein the catalyst is used in a molar ratio of 0.001 to 0.3, based on the amino groups.

6. The process according to any one of claims 1 to 5, wherein the formic acid ester is a linear or branched C₁-C₆-alkyl formate or C₂-C₆-1-alkenyl formate.

7. The process according to claim 6, wherein the formic acid ester is methyl formate.

8. The process according to any one of claims 1 to 7, wherein the formic acid ester is used in a molar ratio of formic acid ester to amino groups of from 1:1 to 20:1.

9. The process according to any one of claims 1 to 8, wherein the aromatic amines are primary or secondary amines of the general formula R²(NHR³)ₙ, in which R is an optionally substituted C₆-C₃₄-aryl radical, R³ is a C₁-C₄-alkyl radical or a hydrogen atom and n is an integer from 1 to 3 per aromatic cycle.

10. The process according to claim 9, wherein the aromatic amines are primary amines.

11. The process according to claim 10, wherein the aromatic amines are selected from the group comprising: aniline, diaminotoluene (TDA), in particular 2,4- and 2,6-diaminotoluene and isomer mixtures thereof, diaminodiphenylmethane (MDA), in particular 2,4'-diaminodiphenylmethane, 4,4'-diaminodiphenylmethane, 2,2'-diaminodiphenylmethane and higher homologues (polyphenylenepolymethylenepolyamines) and mixtures thereof.

12. The process according to any one of claims 1 to 11, wherein the suspension-like reaction discharge is filtered, the formamide being isolated as solid.

13. The process according to claim 12, wherein the catalyst present in the filtrate and any solvents present, excesses of formic acid ester, starting material residues and aminoformamides, individually or separately, are reused and/or recycled in a further reaction cycle.

## Revendications

1. Procédé pour la préparation de formamides par transformation d'amines aromatiques avec un ester de l'acide formique en présence d'un catalyseur, **caractérisé en ce que** le catalyseur est un acide contenant du phosphore ou un sel métallique acide de Lewis d'un acide inorganique ou organique et le métal du sel métallique acide de Lewis est choisi dans le groupe contenant : le zinc, le plomb et l'ytterbium.

2. Procédé selon la revendication 1, **caractérisé en ce que** l'acide contenant du phosphore est l'acide orthophosphorique ou un produit de condensation de poids moléculaire supérieur.

3. Procédé selon la revendication 1, **caractérisé en ce que** le sel métallique acide de Lewis est un carboxylate, un sulfonate ou un nitrate de zinc, de plomb ou d'ytterbium.

4. Procédé selon la revendication 3, **caractérisé en ce que** le sel métallique acide de Lewis est l'acétate de zinc, l'acétate de zinc dihydraté, le naphténate de zinc ou le trifluorométhanesulfonate d'ytterbium.

5. Procédé selon l'une quelconque des revendications 1 à 4, **caractérisé en ce que** le catalyseur est utilisé en un rapport molaire de 0,001 à 0,3 par rapport aux groupes amino.

6. Procédé selon l'une quelconque des revendications 1 à 5, **caractérisé en ce que** l'ester de l'acide formique est un formiate de C₁-C₆-alkyle ou de C₂-C₆-1-alcényle linéaire ou ramifié.

7. Procédé selon la revendication 6, **caractérisé en ce que** l'ester de l'acide formique est le formiate de méthyle.

8. Procédé selon l'une quelconque des revendications 1 à 7, **caractérisé en ce que** l'ester de l'acide formique est utilisé dans un rapport molaire ester d'acide formique à groupes amino de 1:1 à 20:1.

9. Procédé selon l'une quelconque des revendications 1 à 8, **caractérisé en ce que** les amines aromatiques sont des amines primaires ou secondaires de formule générale R²(NHR³)ₙ, R représentant un radical C₆-C₃₄-aryle le cas échéant substitué, R³ représentant un radical C₁-C₄-alkyle ou un atome d'hydrogène et n valant un nombre entier de 1 à 3 par cycle aromatique.

10. Procédé selon la revendication 9, **caractérisé en ce que** les amines aromatiques sont des amines primaires.

11. Procédé selon la revendication 10, **caractérisé en ce que** les amines aromatiques sont choisies dans le groupe contenant : l'aniline, le diaminotoluène (TDA), en particulier le 2,4-diaminotoluène et le 2,6-diaminotoluène et leurs mélanges d'isomères, le diaminodiphénylméthane (MDA), en particulier le 2,4'-diaminodiphénylméthane, le 4,4'-diaminodiphénylméthane, le 2,2'-diaminodiphénylméthane et des homologues supérieurs (polyphénylènepolyméthylènepolyamines) et leurs mélanges.

12. Procédé selon l'une quelconque des revendications 1 à 11, **caractérisé en ce que** le produit de réaction sous forme de suspension est filtré, le formamide étant isolé en tant que solide.

13. Procédé selon la revendication 12, **caractérisé en ce que** le catalyseur et le solvant, les excès d'ester d'acide formique, les restes de produit de départ et les aminoformamides, le cas échéant présents, se trouvant dans le filtrat sont réutilisés ou recyclés, ensemble ou séparément, dans un autre cycle de réaction.
